Europäisches Patentamt

(19) European Patent Office

Office européen des brevets

(11) Veröffentlichungsnummer: **0 127 810**
A1

(12) # EUROPÄISCHE PATENTANMELDUNG

(21) Anmeldenummer: 84105468.7

(22) Anmeldetag: 14.05.84

(51) Int. Cl.³: **C 07 C 59/125**
C 09 D 7/00

(30) Priorität: 21.05.83 DE 3318596

(43) Veröffentlichungstag der Anmeldung:
12.12.84 Patentblatt 84/50

(84) Benannte Vertragsstaaten:
AT BE CH DE FR GB IT LI LU NL SE

(71) Anmelder: Henkel Kommanditgesellschaft auf Aktien
Postfach 1100 Henkelstrasse 67
D-4000 Düsseldorf-Holthausen(DE)

(72) Erfinder: Meffert, Alfred, Dr.
Marie-Curie-Strasse 10
D-4019 Moheim(DE)

(72) Erfinder: Döbrich, Peter
Grupellostrasse 2
D-4000 Düsseldorf 1(DE)

(54) Hydroxyalkoxycarbonsäuren und deren Salze, ihre Herstellung und Verwendung.

(57) Die Erfindung beschreibt neue Hydroxyalkoxycarbonsäuren und deren Salze. Die Produkte werden aus den Epoxiden ungesättigter Fettsäureester durch Ringöffnung und Verseifung mit Alkoholen hergestellt. Sie weisen lösungsvermittelnde Eigenschaften auf und können als Solubilisatoren in wäßrigen Lackzubereitungen eingesetzt werden. Dabei sind die Aminsalze besonders bevorzugt.

EP 0 127 810 A1

0127810

HENKEL KGaA
ZR-FE/Patente
Dr.Wik/Po

Henkelstraße 67

4ooo Düsseldorf, den 18.5.1983

BEZEICHNUNG GEÄNDERT
Siehe Titelseite

P a t e n t a n m e l d u n g

D 6847 EP .

"Neue Hydroxyalkoxycarbonsäuren und deren Salze, ihre
Herstellung und Verwendung"

Die Erfindung liegt auf dem Gebiet der Oleochemie. Sie
betrifft neue nützliche Folgeprodukte natürlich vorkommender ungesättigter Fettsäuren. Es ist bekannt, daß
Fettsäureester z.B. Methylester aber auch Glycerinester
mit Persäuren, insbesondere mit Peressigsäure zu Epoxyden umgesetzt werden können, wobei die Epoxidgruppe
anstelle der Doppelbindung tritt. Ein entsprechendes
Verfahren wird beispielsweise von T.W. Findley et al.
in J. Am. Chem. Soc. 67, 412 (1945) beschrieben.

Die epoxydierten Fettsäureester können mit Alkoholen
in Gegenwart von Säuren unter Ringöffnung und Umesterung in Ester der Hydroxyalkoxycarbonsäuren überführt werden. Dazu kann nach dem Verfahren von A. Bilyk
et al., beschrieben in J. Am. Oil Chem. Soc. 51, 119,
(1974) gearbeitet werden.

Obwohl zahlreiche Ester von Hydroxyalkoxycarbonsäuren
insbesondere von Hydroxyalkoxyfettsäuren bekannt und
beschrieben sind, finden sich in der Literatur keine
Hinweise auf die Säuren selbst oder deren Salze.

...

Dies mag daran liegen, daß aufgrund der Hydroxyl- und Alkoxygruppen im hydrophoben Molekülteil die Fachwelt keine interessanten Eigenschaften, insbesondere keine Tensideigenschaften erwartet hat. Es wurde nun überraschend gefunden, daß langkettige Hydroxyalkoxycarbonsäuren und insbesondere deren Aminsalze nützliche Hilfsstoffe bei der Konfektionierung von Alkydharzen zu Wasserlacken sind.

Aufgabe der Erfindung ist es somit, auf Basis natürlicher nachwachsender Rohstoffe neue Carbonsäuren mit 8 - 24 C-Atomen bereitzustellen, die Hydroxy- und Alkoxysubstituenten tragen, sowie deren Salze. Eine weitere Aufgabe der Erfindung ist es, ein Verfahren zur Herstellung dieser Säuren aus den Derivaten natürlich vorkommender ungesättigter Fettsäuren aufzuzeigen.

Gegenstand der Erfindung sind somit in einer ersten Ausführungsform unverzweigte Carbonsäuren mit einer geraden Anzahl an Kohlenstoffatomen zwischen 8 und 24, dadurch gekennzeichnet, daß sie innenständig an benachbarten Kohlenstoffatomen Hydroxylgruppen und Alkoxygruppen aufweisen (Hydroxyalkoxycarbonsäuren), welche durch Epoxidation einer olefinischen Doppelbindung und anschließende Ringöffnung mit Alkoholen eingeführt worden sind, sowie deren Salze.

Ein weiterer Gegenstand der Erfindung ist ein Verfahren zur Herstellung von Hydroxyalkoxycarbonsäuren, dadurch gekennzeichnet, daß Ester ungesättigter Fettsäuren epoxydiert, die Epoxide unter Säurekatalyse mit einem Überschuß eines aliphatischen Alkohols unter Ring-

...

öffnung und ggf. Umesterung umgesetzt werden, die Reaktionsmischungen sodann bei Temperaturen zwischen 20 und 60$^{\circ}$ mit Alkalihydroxyden versetzt werden und anschließend bei Temperaturen zwischen 80 und 110$^{\circ}$ zu den Hydroxyalkoxycarbonsäuren verseift werden.

Die erfindungsgemäßen Hydroxyalkoxycarbonsäuren leiten sich von natürlich vorkommenden Fettsäuren ab. Sie weisen daher eine gerade Anzahl an Kohlenstoffatomen in der Hauptkette auf und sind nicht verzweigt. Für technische Verwendungen werden natürliche Fettsäuren meistens als Mischungen eingesetzt. Diese Mischungen enthalten gesättigte, einfach ungesättigte und mehrfach ungesättigte Fettsäuren. Auch bei den erfindungsgemäßen Hydroxyalkoxycarbonsäuren ist es bevorzugt, Mischungen unterschiedlicher Kettenlänge einzusetzen, die auch noch gesättigte Anteile oder aber Hydroxyalkoxycarbonsäuren mit Doppelbindungen enthalten können.

In einer ersten bevorzugten Ausführungsform leiten sich die erfindungsgemäßen Hydroxyalkoxycarbonsäuren von einfach ungesättigten Fettsäuren ab. So sind 9,10-Hydroxyalkoxypalmitinsäure, 9,10-Hydroxyalkoxystearinsäure und 13,14-Hydroxyalkoxybehensäure sowie deren 10,9 bzw. 14,13-Isomere besonders bevorzugte Produkte im Sinne der Erfindung.

In einer weiteren Ausführungsform leiten sich die erfindungsgemäßen Hydroxyalkoxycarbonsäuren von mehrfach ungesättigten Fettsäuren ab. Bevorzugt sind die Folgeprodukte mehrfach ungesättigter Fettsäuren mit 18 C-Atomen. Derartige Hydroxycarbonsäuren sind beispielsweise $\Delta^{12}$-9,10-Hydroxyalkoxyoctadecensäure, $\Delta^9$-12,13-Hydroxyalkoxyoctadecensäure, $\Delta^{12,15}$-9,10-Hydroxyalkoxyoctadecadiensäure sowie deren Isomere mit der Hydroxyalkoxygruppe an den Kohlenstoffatomen 12,13 bzw. 15,16. Diese ungesättigten Hydroxyalkoxycarbonsäuren leiten sich von Linol- bzw. Linolensäure ab, wobei nur eine Doppelbindung durch Epoxidation und anschließende Ringöffnung umgesetzt worden ist. Weitere Hydroxyalkoxycarbonsäuren im Sinne der Erfindung sind die Umsetzungsprodukte von Linol- und Linolensäure, bei denen 2 oder alle Doppelbindungen epoxydiert und anschließend zu Hydroxyalkoxygruppen umgesetzt worden sind.

In einer weiteren Ausführungsform betrifft die Erfindung Hydroxyalkoxycarbonsäuren, die sich von weniger häufigen ungesättigten Fettsäuren herleiten. So können Hydroxyalkoxycarbonsäuren hergestellt werden aus $\Delta^9$-Decylensäure, Stilingasäure, $\Delta^9$-Dodecylensäure, Rizinolsäure, Petroselinsäure, Vaccensäure, Eläostearinsäure, Punicinsäure, Licansäure, Parinarsäure, Gadoleinsäure, Arachidonsäure, 5-Eicosensäure, 5-Docosensäure, Cetoleinsäure, 5,13-Docosadiensäure und/oder Selacholeinsäure.

In einer weiteren Ausführungsform betrifft die Erfindung Hydroxyalkoxycarbonsäuren, die aus Isomerisierungsprodukten natürlicher ungesättigter Fettsäuren hergestellt worden sind. Die so hergestellten Hydroxyalkoxy-

...

- 5 -

carbonsäuren unterscheiden sich nur durch die Lage der Hydroxy- und der Alkoxygruppe im Molekül. Sie liegen im allgemeinen als Gemische vor.

Der Alkoxyrest der Hydroxyalkoxycarbonsäuren leitet sich von dem Alkohol ab, der zur Ringöffnung des epoxydierten Fettsäurederivats verwendet worden ist. Nach einer bevorzugten Ausführungsform der Erfindung werden Hydroxyalkoxycarbonsäuren beansprucht, deren Alkoxygruppe sich von primären monofunktionellen Alkoholen mit bis zu 6 C-Atomen ableitet. Geeignete Alkohole sind Methanol, Ethanol, Propanol, Butanol, Pentanol und Hexanol. Eine weitere Ausführungsform der Erfindung betrifft Hydroxyalkoxycarbonsäuren deren Alkoxygruppe sich von sekundären    Alkoholen mit bis zu 6 C-Atomen ableitet, beispielsweise von Isopropanol, Isobutanol, von $C_5$- oder $C_6$-Alkoholen mit innenständiger OH-Gruppe von cyclischen $C_5$- oder $C_6$-Alkoholen oder von verzweigten aliphatischen Alkoholen mit bis zu 6 C-Atomen.

Eine weitere Ausführungsform der Erfindung betrifft Hydroxyalkoxycarbonsäuren deren Alkoxyrest sich von Alkoholen ableitet, die eine weitere funktionelle Gruppe tragen. So kann sich der Alkoxyrest z.B. von Monoalkylethern des Ethylenglykols oder Propylenglykols mit bis zu 3 C-Atomen in der Alkylgruppe ableiten.

Eine weitere Ausführungsform der Erfindung betrifft Hydroxyalkoxycarbonsäuren deren Alkoxyrest sich von

...

- 6 -

mehrfunktionellen Alkoholen ableitet. Derartige Produkte liegen im allgemeinen im Gemisch mit mehr- oder mindergrossen Mengen an Mehrfachumsetzungsprodukten vor. Mehrfachumsetzungsprodukte sind Hydroxyalkoxycarbonsäuren, deren Alkoxyrest eine Brücke zwischen 2 oder mehr Fettsäure-Molekülen bildet. Es ist dem Fachmann bekannt, daß die Menge derartiger Mehrfachsubstitutionsprodukte durch eine gezielte Wahl der Reaktionsbedingungen zurückgedrängt werden kann. So werden Hydroxyalkoxycarbonsäuren, die freie Hydroxylgruppen am Alkoxyrest tragen, bevorzugt in der Weise hergestellt, daß das epoxydierte Fettsäurederivat unter Reaktionsbedingungen zu einem hohem Überschuß an mehrfach ungesättigtem Alkohol zugegeben wird. Bevorzugte erfindungsgemäße Hydroxyalkoxycarbonsäuren, die am Alkoxyrest freie Hydroxylgruppen tragen, werden erhalten, indem man epoxydierte Fettsäureester mit einem hohen Überschuß der folgenden Alkohole umsetzt: Ethylenglykol, Propylenglykol, Diethylenglykol, Dipropylenglykol, Glycerin, Trimethylolethan, Trimethylolpropan oder Pentaerythrit. Bevorzugt wird mit einem Überschuß von 1/2 mol bis zu mehr als 10 mol Alkohol/mol epoxydierten Fettsäurederivats gearbeitet.

Zur Herstellung der erfindungsgemäßen Hydroxyalkoxycarbonsäuren werden epoxydierte Carbonsäureester, z.B. epoxydierte Fettsäuremethyl-, -ethyl-, -propyl- oder -glycerinester mit den Alkoholen, von denen sich die Alkoxygruppe ableiten soll, unter Ringöffnungs- und gewünschtenfalls Umesterungsbedingungen umgesetzt. Dazu können bekannte Verfahren herangezogen werden. Es ist bevorzugt, den zur Umsetzung vorgesehenen Al- kohol zusammen mit einem sauren Katalysator etwa einer starken Mineralsäure vorzulegen und bei einer Reaktionstemperatur zwischen 80 und 120° das epoxydierte Fettsäurederivat kontinuierlich oder portionsweise zuzugeben. Das Fortschreiten der Reaktion kann durch Titration des Restepoxidsgehalt oder mittels spektroskopischen Methoden überwacht werden. Wenn alle Epoxidgruppen umgesetzt sind, wird der saure Katalysator durch Neutralisation zerstört. Die so entstandenen Hydroxyalkoxycarbonsäureester können destillativ von überschüssigem Alkohol befreit werden.

In einer zweiten Stufe wird dann die Verseifung der Hydroxyalkoxycarbonsäureester zu den Hydroxyalkoxycarbonsäuren durchgeführt. Die Verseifung wird vorzugsweise bei Temperaturen zwischen 40 und 120° in Gegenwart von Wasser unter basischer Katalyse durchgeführt. Geeignete Basen sind die Hydroxide der Alkali- und/oder Erdalkalimetalle weiterhin tertiäre Amine. Die Hydroxyalkoxycarbonsäuren fallen nach dieser Reaktionsstufe als Salze (Seifen) an und können durch Versetzen mit starken Säuren z.B. Salzsäure oder Schwefelsäure gewonnen werden. Dabei ist es möglich, die Reaktionsprodukte durch einfaches oder gewünschtenfalls mehrfaches Waschen mit Wasser zu reinigen.

Die Salze der Hydroxyalkoxycarbonsäuren können entweder direkt bei der Verseifung durch Auswahl eines geeigneten Verseifungsmittels etwa des Hydroxids oder Carbonats eines bestimmten Kations hergestellt werden oder aber können sie aus den freien Säuren durch Zugabe eines Hydroxids oder eines Amins erhalten werden. Aufgrund ihrer Wasserlöslichkeit sind für viele Anwendungen die Salze

...

der Hydroxyalkoxycarbonsäuren mit monofunktionellen Kationen bevorzugt, insbesondere die Lithium-, Natrium Kaliumsalze. Weitere bevorzugte Salze sind die Salze der Hydroxyalkoxycarbonsäuren mit Ammoniak oder primären, sekundären oder tertiären Aminen. So können Ammoniumsalze, Methylammonium-, Dimethylammonium-, Trimethylammonium- etc. Salze hergestellt werden.

Eine besonders wichtige Untergruppe der Aminsalze sind die Salze der Hydroxyalkoxycarbonsäuren mit Aminoalkoholen wie z.B. Dimethylethanolamin, Triethanolamin, 2-Methyl-3-aminopropanol, 2-Methyl-2-aminopropandiol sowie Aminoalkohole, die durch Addition von Glycid an primäre oder sekundäre Amine herstellbar sind.

Die so hergestellten Salze der Hydroxyalkoxycarbonsäuren mit monovalenten Kationen sind wasserlöslich. Sie haben die Fähigkeit, ansich wasserunlösliche oder schwer wasserlösliche Substanzen in wäßriger Lösung zu halten. Dies trifft insbesondere auf die Salze mit Aminoalkoholen, besonders mit mehrfach OH-funktionellen Aminoalkoholen zu.

Aufgrund dieser Eigenschaften eignen sich die Salze aber im beschränkten Maße auch die freien Säuren als Konfektionierungshilfsmittel für Alkydharze zur Herstellung von ofentrocknenden Wasserlacken. Die neuen Verbindungen haben dabei insbesondere den Vorteil, daß sie unter Einbrennbedingungen in das Harz eingebaut werden, wobei ihre Hydrophilie zumindest teilweise durch Verbrauch an hydrophilen Gruppen verschwindet. Zwar können sowohl Wasserlacke mit sehr hoher Säurezahl, z.B. mit Säurezahlen zwischen 50 und 80 wie auch solche

. . .

- 9 -

mit relativ niederer Säurezahl, das sind Säurezahlen
zwischen 50 und 15 konfektioniert werden. Bevorzugt
ist die Verwendung der erfindungsgemäßen Hydroxyalkoxycarbonsäuresalze jedoch im Bereich niederer Säurezahlen,
insbesondere zur Solubilisierung von Harzen mit einer
Säurezahl von nur 20 - 30 oder sogar von nur 10 - 20.
Die Verwendung der Produkte  führt dabei zu für das
Auge klaren, wäßrigen Zubereitungen, die auch bei Erwärmen z.B. auf 60 - 100° keine Phasentrennung zeigen.
Die zur Solubilisierung von Alkydharzen benötigten
Mengen an Hydroxyalkoxycarbonsäuren richten sich in
erster Linie nach der Säurezahl. In den meisten Fällen ist es jedoch ausreichend, bezogen auf Harz,5 -
15 % der Salze zu verwenden.

...

### B e i s p i e l e

1. Umsetzung von epoxydierten Fettsäureestern mit
   Alkoholen (Gewichtsmengen in Tabelle I)

   Alkohol und Schwefelsäure wurde im Reaktionsgefäß vorgelegt und auf Reaktionstemperatur von
   90°C gebracht, danach ließ man den epoxydierten
   Fettsäureester während 1 Stunde zulaufen und während einer weiteren Stunde abreagieren. Danach
   wurde auf ca. 50°C gekühlt und mit Natriummethylat
   neutralisiert. Die Kennzahlen der so entstandenen
   Epoxyalkoxyfettsäureester sind in Tabelle I zusammengefaßt.

. . . .

D 6847 EP

Ringöffnung der epoxydierten Fettsäurederivate mit Alkoholen

| Nr.: | Epoxy.Fett-säuremethyl-ester[+)] (g) | Epox.So-jaöl Epoxid-zahl: 6,1 (g) | $H_2SO_4$ (g) | Methanol (g) | Ethylen-glykol (g) | Trimethylol-propan (g) | Glycerin (g) | Reaktions-temperatur | OH-Zahl (mgKOH/g | Versei-fungs-zahl |
|---|---|---|---|---|---|---|---|---|---|---|
| 1 | 3868 | | 8,8 | 2112 | | | | 65 | 146,5 | 170,8 |
| 2 | 3868 | | 2,2 | | 2046 | | | 80 | 252,1 | 158,7 |
| 3 | 3868 | | 1,65 | | | 2952 | | 90 | 343,8 | 130,4 |
| 4 | 3868 | | 3,3 | | | | 2024 | 90 | 299,0 | 153,6 |
| 5 | | 3184 | 7,0 | 2374 | | | | 65 | 204,2 | |
| 6 | | 3184 | 3,6 | | 2232 | | | 90 | 230,7 | |
| 7 | | 3184 | 2,4 | | | 3221 | | 95 | 352,5 | |

...

## Fortsetzung T A B E L L E I

Erläuterung:

+) Epoxid eines Ölsäuremethylesters der
folgenden Kettenlängenverteilung (Gew.-%)

| | | | | |
|---|---|---|---|---|
| 0 | – | 2 | % | $C_{12}$ |
| 2 | – | 5 | % | $C_{14}$ |
| 5 | – | 7 | % | $C_{16}$ |
| 5 | – | 7 | % | $C_{16}$ einfach ungesättigt |
| 1 | – | 3 | % | $C_{18}$ |
| 70 | – | 75 | % | $C_{18}$ einfach ungesättigt |
| 8 | – | 12 | % | $C_{18}$ zweifach ungesättigt |
| 0 | – | 1 | % | $C_{18}$ dreifach ungesättigt |
| 0 | – | 4 | % | höhere |

Epoxidzahl: 4,55 Gew.-% Epoxidsauerstoff

0127810 HENKEL KGaA ZR-FE/Patente

2. Herstellung der Hydroxyalkoxycarbonsäuren

Die Produkte der Beispiele 1 - 7 aus Tabelle I wurden bei 40$^\circ$C mit einem Überschuß wässriger Natriumhydroxydlösung versetzt (siehe Tabelle II) und während 3 Stunden bei 90 - 100$^\circ$C belassen. Nach vollendeter Verseifung wurde auf 70$^\circ$C abgekühlt und mit konzentrierter Salzsäure sauer gestellt (pH = ca. 2). Die wässrige Phase wurde heiß abgetrennt und das Produkt zweimal mit 60$^\circ$C warmen Wasser gewaschen, anschließend im Vakuum getrocknet. Kennzahlen sind in Tabelle II aufgelistet.

. . .

Patentanmeldung    D 6847 EP

# T A B E L L E   II

Verseifung der Hydroxyalkoxycarbonsäureester

Einsatzmengen und Kennzahlen

| Produkt | Hydroxyalkoxy-carbonsäureester (g) | NaOH (g) | $H_2O$ (g) | OH - Zahl (mgKOH/g) | Säurezahl (mg KOH/g) |
|---|---|---|---|---|---|
| 1 | 2628 | 480 | 1200 | 145,3 | 170,9 |
| 2 | 2430 | 420 | 1400 | 250,6 | 158,0 |
| 3 | 2602 | 360 | 1400 | 323,5 | 130,2 |
| 4 | 2192 | 360 | 1400 | 288,1 | 147,5 |
| 5 | 2473 | 540 | 1500 | 169,1 | 159,7 |
| 6 | 3312 | 409 | 2000 | 224,2 | 157,0 |
| 7 | 3890 | 489 | 2500 | 332,1 | 114,9 |

0127810

HENKEL KGaA
ZR-FE/Patente

...

<u>Patentansprüche</u>

1. Unverzweigte Carbonsäuren mit einer geraden Anzahl an Kohlenstoffatomen zwischen 8 und 24, dadurch gekennzeichnet, daß sie innenständig an benachbarten Kohlenstoffatomen Hydroxylgruppen und Alkoxygruppen aufweisen (Hydroxyalkoxycarbonsäuren), welche durch Epoxidation einer olefinischen Doppelbindung und anschließende Ringöffnung mit Alkoholen eingeführt worden sind, sowie deren Salze.

2. Hydroxyalkoxycarbonsäuren nach Anspruch 1, dadurch gekennzeichnet, daß es sich um Hydroxyalkoxycaprinsäuren, -laurinsäuren, -myristinsäuren, -palmitinsäuren, -stearinsäuren, -octadecensäuren, -arachinsäuren, -behensäuren und/oder -lignocerinsäuren handelt.

3. Hydroxyalkoxycarbonsäuren nach den Ansprüchen 1 und 2, dadurch gekennzeichnet, daß es sich bei dem Alkoxyrest um eine über ein Sauerstoffatom gebundene lineare oder verzweigte Alkylgruppe mit bis zu 6 C-Atomen handelt, die mit bis zu 3 Hydroxylgruppen und/oder einer Alkoxygruppe mit bis zu 3 C-Atomen substituiert sein kann.

4. Hydroxyalkoxycarbonsäuren nach Anspruch 3, dadurch gekennzeichnet, daß sich die Alkoxygruppe von den Alkoholen Methanol, Ethanol, Etylenglykol, Propylenglykol, Diethylenglykol, Dipropylenglykol, Glycerin, Trimethylolethan, Trimethylolpropan und/oder Pentaerythrit ableitet.

...

5. Salze von Hydroxyalkoxycarbonsäuren nach den Ansprüchen 1 - 4, dadurch gekennzeichnet, daß sie als Gegenion Natrium, Kalium, Ammonium, Alkylammonium, Dialkylammonium, Trialkylammonium und/oder insbesondere eine Ammoniumgruppe, die sich von einem mehrfach OH-funktionellen Aminoalkohol ableitet, aufweisen.

6. Verfahren zur Herstellung von Hydroxyalkoxycarbonsäuren nach den Ansprüchen 1 - 5, dadurch gekennzeichnet, daß Ester ungesättigter Fettsäuren epoxydiert, die Epoxide unter Säurekatalyse mit einem Überschuß eines aliphatischen Alkohols unter Ringöffnung und gegebenenfalls Umesterung umgesetzt werden, die Reaktionsmischungen sodann bei Temperaturen zwischen 20 und 60° mit Alkalihydroxyden und/oder Aminen versetzt werden und anschließend bei Temperaturen zwischen 80 und 110° zu den Salzen der Hydroxyalkoxycarbonsäuren verseift werden, welche gewünschtenfalls in die freien Säuren umgewandelt werden können.

7. Verwendung der Hydroxyalkoxycarbonsäuren und/oder ihrer Salze nach den Ansprüchen 1 - 5 als Emulgatoren und/oder Solubilisatoren in wäßrigen Lackzubereitungen, insbesondere zur Konfektionierung von Alkydharzen mit Säurezahlen zwischen 10 und 80, vorzugsweise 35 bis 50.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | EP-A-0 022 236 (HENKEL KGaA) <br> * Ansprüche 1-4; Seite 7, Zeile 28 - Seite 8, Zeile 1, Zeilen 13-21; Seite 11, Zeilen 12-17 * <br><br> --- | 1-3,6 | C 07 C 59/125 <br> C 09 D 7/00 |
| A | US-A-3 119 848 (WRIGLEY u.a.) <br> * Anspruch 1 * <br><br> --- | 1 | |
| A | DE-A-2 256 908 (HENKEL KGaA) <br> * Anspruch * <br><br> ----- | 1 | |

**RECHERCHIERTE SACHGEBIETE (Int. Cl. ³)**

C 07 C 59/00

Der vorliegende Recherchenbericht wurde für alle Patentansprüche erstellt.

| Recherchenort <br> DEN HAAG | Abschlußdatum der Recherche <br> 23-08-1984 | Prüfer <br> KLAG M.J. |
|---|---|---|